# EUROPÄISCHE PATENTSCHRIFT

(11) **EP 0 584 118 B1**
(45) Veröffentlichungstag und Bekanntmachung des Hinweises auf die Patenterteilung: **27.09.2000**
(21) Anmeldenummer: 92909423.3
(22) Anmeldetag: 01.05.1992
(51) Int. Cl.: A61K 47/48

(54) **NEUE KONJUGATE, BESTEHEND AUS EINEM GLYKOPROTEIN UND EINER NUKLEINSÄURE-BINDENDEN SUBSTANZ**
NEW CONJUGATES, CONSISTING OF A GLYCOPROTEIN AND A NUCLEIC ACID-BINDING SUBSTANCE
NOUVEAUX CONJUGUES COMPOSES D'UNE GLYCOPROTEINE ET D'UNE SUBSTANCE LIANT DES ACIDES NUCLEIQUES

(30) Priorität: 08.05.1991 DE 4115038
(43) Veröffentlichungstag der Anmeldung: 02.03.1994
(73) Patentinhaber: GENENTECH, INC., South San Francisco California 94080 (US); Boehringer Ingelheim International GmbH, 55218 Ingelheim am Rhein (DE)
(72) Erfinder: BIRNSTIEL, Max, L., A-1100 Wien (AT); COTTEN, Matthew, A-1130 Wien (AT); WAGNER, Ernst, A-2103 Langenzersdorf (AT)
(74) Vertreter: Laudien, Dieter, Dr.
(86) Internationale Anmeldenummer: EP9200953
(87) Internationale Veröffentlichungsnummer: WO9219281

(56) Entgegenhaltungen:
- EP-A- 0 306 473
- EP-A- 0 388 758
- WO-A-79/00515
- WO-A-88/05077
- WO-A-91/17773
- Proceedings of the National Academy of Sciences, of the United States of America, Band 87, Nr. 9, Mai 1990, Biochemistry, E. WAGNER et al.: "Transferrin-polycation conjugates as carriers for DNA uptake into cells", Seiten 3410-3414, siehe Zusammenfassung
- The Journal of Biological Chemistry, Band 262, Nr. 10, 5. April 1987, The American Society of Biological Chemists, Inc., (US), G.Y. WU et al.: "Receptor-mediated in vitro gene transformation by a soluble DNA carrier system", Seiten 4429-4432
- Bioconjugate Chem., Band 2, 1991, American Chemical Society, E. WAGNER et al.: "DNA-binding transferrin conjugates as functional gene-delivery agents: synthesis by linkage of polylysine or ethidium homodimer the transferrin carbohydrate moiety", Seiten 226-231, siehe Figur 1
- STN File Server, Base Biosis, AN=91:341061, G.Y. WU et al.: "Delivery systems for gene therapy", & BIOTHERAPY (DORDR.), 3(1), 1991, 87-96, siehe Zusammenfassung

## Beschreibung

Die vorliegende Erfindung bezieht sich auf Konjugate, bestehend aus einem Glykoprotein und einer Nukleinsäure-bindenden Substanz, die befähigt sind, mit Nukleinsäuren oder Nukleinsäureanalogen in höhere eukaryotische Zellen aufnehmbare Komplexe zu bilden, sowie auf Verfahren zu deren Herstellung.

Die europäische Patentanmeldung EP A1 0388 758 beschreibt Transferrin-Polykation-Konjugate, die befähigt sind, mit Nukleinsäure Komplexe zu bilden. Diese Komplexe werden effizient in lebende Zellen internalisiert und die importierte DNA exprimiert. Die Aufnahme der Transferrin-Polykation-DNA-Komplexe in die Zelle erfolgt über den Transferrinrezeptor.

In der WO 91/17773 werden Konjugate aus einem Protein mit der Fähigkeit, an CD4 zu binden, und Polykationen beschrieben, die für den Transport von Nukleinsäuren in CD4 esprimierende Zellen verwendet werden können. Zu geeigneten CD4 bindenden Proteinen zählt das virale Glykoprotein gp120.

Ein in der EP A1 0388 758 und in der WO 91/17773 beschriebenes Verfahren zur Herstellung der Protein-Polykation-Konjugate besteht darin, Transferrin bzw. das CD4 bindende Protein und das Polykation auf chemischem Weg zu koppeln, wobei es sich um bekannte Methoden zur Kopplung von Peptiden handelt. Bei derartigen Methoden werden im allgemeinen die Einzelkomponenten vor der Kopplungsreaktion mit Linkersubstanzen versehen (diese Maßnahme ist dann erforderlich, wenn von vornherein keine für die Kopplung geeignete funktionelle Gruppe, z.B. eine Mercapto- oder Alkoholgruppe, verfügbar ist. Bei den Linkersubstanzen handelt es sich um bifunktionelle Verbindungen, die zunächst mit funktionellen Gruppen der Einzelkomponenten zur Reaktion gebracht werden, worauf die Kopplung der modifizierten Einzelkomponenten durchgeführt wird).
Je nach gewünschten Eigenschaften der Konjugate, insbesondere im Hinblick auf deren Stabilität, kann bei diesen Methoden unter Verwendung von Linkersubstanzen die Kopplung erfolgen über
a) Disulfidbrücken, die unter reduzierenden Bedingungen wieder gespalten werden können (z.B. bei Verwendung von Succinimidylpyridyldithiopropionat (Jung et al., 1981).
b) Unter biologischen Bedingungen weitgehend stabile Verbindungen (z.B. Thioether durch Reaktion von Maleimido-Linkern mit Sulfhydrylgruppen des an die zweite Komponente gebundenen Linkers).
c) Unter biologischen Bedingungen labile Brücken, z.B. Esterbindungen, oder unter schwach sauren Bedingungen instabile Acetal- oder Ketalbindungen.

Bei der Synthese wird auf Grund der weitgehend statistischen Verteilung der Linkergruppen über das Proteinmolekül ein Gemisch von Konjugaten mit unterschiedlichem Protein/Polykation-Verhältnis erhalten, das erst in homogene Fraktionen aufgetrennt werden muß, wodurch sich solche Verfahren zur Herstellung größerer Konjugatmengen nicht eignen. Außerdem weisen diese Verfahren den Nachteil auf, daß die erhaltenen Konjugate hinsichtlich der Stelle der Bindung zwischen dem Protein und den Nukleinsäurebindenden Substanzen nicht exakt definiert sind. Dabei kann nicht ausgeschlossen werden, daß das Polykation an einer Stelle des Glykoproteins bindet, deren Zugänglichkeit für die Bindung des Glykoproteins an das Zelloberflächenprotein verantwortlich ist, wodurch die Internalisierung beeinträchtigt werden kann. Ein weiterer Nachteil der nach solchen Methoden hergestellten Konjugate besteht darin, daß aufgrund der im Überschuß eingesetzten Linkersubstanzen, die nicht alle für die Kopplung benutzt werden, Linkerreste auf dem Molekül verbleiben, die gegebenenfalls eine Veränderung der biologischen Aktivität des Proteins hervorrufen, die die Wirksamkeit der Konjugate nachteilig beeinflußt.

Der vorliegenden Erfindung lag die Aufgabe zugrunde, verbesserte Konjugate, bestehend aus einem Glykoprotein und einer Verbindung mit der Fähigkeit, Nukleinsäure zu binden, sowie ein Verfahren zur Herstellung solcher Konjugate bereitzustellen.

Das humane Transferrin ist hinsichtlich seiner Aminosäuresequenz (MacGillivray et al., 1983), der Glykosylierungsstellen und der Struktur der Kohlenhydratseitenketten (Dorland et al., 1977) aufgeklärt. Das Protein enthält zwei identische Kohlenhydratketten, die durch N-glykosydische Bindung an Asn-413 und Asn-611 gebunden sind. Die Glykanketten haben biantennäre Struktur, bestehend aus einem Mannotriosido-di-N-Acetyl-Chitobiose-Kern, der zwei N-Acetylneuraminyl-N-Acetyllactosamin-Gruppen trägt. Soweit bekannt ist, hat die Glykosylierung des Transferrins keinen Einfluß auf die Bindung an den Rezeptor oder auf eine andere biologische Funktion, abgesehen von der Ausscheidung des Asialotransferrins aus dem Plasma (Huebers und Finch, 1987).

gp120 ist das äußere Hüll-Glykoprotein des HIV-Virus. Es weist einen hohen Kohlenhydratanteil auf und ist an der Außenseite der Membran infizierter Zellen und von Viruspartikeln vorhanden. gp120 enthält die für die Bindung des Virus an den CD4-Rezeptor wesentliche Determinante. Die hochaffine Bindung zwischen dem Virus und der Zellmembran wird durch Wechselwirkung zwischen einem Abschnitt von 40 Aminosäuren am C-Terminus von gp120 und einer Domäne nahe dem N-Terminus von CD4 vermittelt. Wenn auch zwischen den verschiedenen HIV-Stämmen erhebliche Unterschiede in den gp120 Sequenzen bestehen, sind die CD4-Bindungsdomänen zwischen HIV-1, HIV-2 und den verwandten SIV-Viren konserviert. Es wurden proteolytische Fragmente von 95 und 25 kDa isoliert, die offensichtlich domänenartige Unterteilungen von gp120 darstellen und an CD4 in gleicher Weise zu binden vermögen wie das ursprüngliche gp120 (Nygren et al., 1988). Die Entfernung der terminalen Sialinsäurereste von der reifen sekretierten Form von gp120 beeinträchtigt nicht die Fähigkeit von gp120, an CD4 zu binden (Fennie und Lasky, 1989).

Van Lenten und Ashwell (1971) beschreiben eine allgemeine Methode zur Markierung von Sialinsäure enthaltenden Glykoproteinen. Dabei werden bei niedrigen Temperaturen die Sialinsäuren der Kohlenhydratkette, die ein vicinales Diolsystem aufweisen, mit Natriumperjodat oxidiert. Ähnliche Versuche wurden mit Transferrin durchgeführt, bei dem die beiden terminalen exozyklischen Kohlenstoffatome der Sialinsäuren in den Kohlenhydratketten durch Perjodatoxidation selektiv entfernt wurden, was durch anschließende Markierung durch Tritiumeinbau in die modifizierten Sialinsäurereste bestätigt wurde (Kishimoto und Tavassoli, 1986).

Morell et al., 1966, beschreiben eine Methode zur Markierung von Ceruloplasmin, wobei zunächst Sialinsäure durch Neuraminidase entfernt und das erhaltene Asialoceruloplasmin mit Galaktoseoxidase behandelt und der entstehende Aldehyd mit Tritiummarkiertem Borhydrid reduziert wird.

Bei der Herstellung von Affinitätschromatographie-Säulen wird für bestimmte Anwendungsfälle die Verbindung zwischen einer Säulenmatrix, die Aminogruppen enthält (entweder von sich aus oder durch Anfügen eines Spacers mit einem terminalen Amin) und dem Antikörper über die Kohlenhydrate auf der schweren Kette hergestellt (Harlow und Lane, 1988).

O'Shannessy und Hoffman, 1987, beschreiben die Herstellung und Verwendung von festen Trägern, die Hydrazid-Funktionen enthalten, für die Immobilisierung von Glykoproteinen, die spezifisch durch die Oligosaccharid-Gruppen erfolgt, wobei das oxidierte Glykoprotein mit dem Hydrazid-derivatisierten Agaroseträger reagieren gelassen wird.

Zur Lösung der im Rahmen der vorliegenden Erfindung gestellten Aufgabe wurde von der Überlegung ausgegangen, die Kohlenhydratgruppen von Glykoproteinen als spezifische Bindungsstellen für Polykationen oder andere nukleinsäurebindende Moleküle heranzuziehen, unter der Voraussetzung, daß die Bindungsfähigkeit des Glykoproteins an die Zelloberfläche durch diese Modifikation nicht beeinträchtigt wird.

Es wurde überraschend festgestellt, daß mit Hilfe von Glykoprotein-Polykation-Konjugaten, in denen die Verbindung zwischen Glykoprotein und Nukleinsäurebindendender Substanz über die Kohlenhydratkette erfolgt, die Effizienz des Transfektionsvorganges gegenüber den nach herkömmlichen Methoden hergestellten Konjugaten gesteigert werden kann.

Die vorliegende Erfindung betrifft somit neue Konjugate mit der Fähigkeit, mit Nukleinsäuren oder Nukleinsäureanalogen Komplexe zu bilden. Die Konjugate bestehen aus einem Glykoprotein und einer Nukleinsäurebindenden Substanz und sind dadurch gekennzeichnet, daß das Glykoprotein und die Nukleinsäure-bindende Substanz über eine oder mehrere Kohlenhydratketten des Glykoproteins miteinander verbunden sind. (Die Nukleinsäure-bindende Substanz wird im folgenden aufgrund ihrer Fähigkeit, die Verbindung zwischen dem Glykoprotein und der Nukleinsäure herzustellen, als "Verbindungsfaktor" bezeichnet.)

Bei den im Rahmen der vorliegenden Erfindung durchgeführten Versuchen wurden die zwei terminalen exozyklischen Kohlenstoffatome der Sialinsäuren in den Kohlenhydratketten von humanem Transferrin selektiv entfernt. Die unter Spaltung der HOC-COH-Bindung erhaltene Aldehyd-Form von Transferrin wurde daraufhin zur Kopplung an die Aminogruppen eines Polykations verwendet. Die aus der Bildung von Aldimin resultierende Bindung wurde zur entsprechenden Aminbindung, die nicht mehr hydrolysierbar ist, reduziert. Die erhaltenen Transferrin-Polykation-Konjugate wurden gereinigt und auf ihre Fähigkeit untersucht, nach Komplexierung mit DNA in lebende Zellen aufgenommen zu werden.

In analoger Weise wurden Konjugate aus gp120 und Polylysin hergestellt und diese Konjugate nach Komplexierung mit DNA auf ihre Fähigkeit untersucht, in CD4⁺-Zellen aufgenommen und exprimiert zu werden.

Im Rahmen der vorliegenden Erfindung konnte gezeigt werden, daß diese Konjugate befähigt sind, mit Nukleinsäure Komplexe zu bilden. Diese können verwendet werden, um therapeutisch wirksame Nukleinsäuren in höhere eukaryotische Zellen einzuführen. Es konnte gezeigt werden, daß die Aufnahme der Glykoprotein-Verbindungsfaktor/Nukleinsäure-Komplexe über den Transferrin-Rezeptor erfolgt.

Die erfindungsgemäßen Konjugate sind im Gegensatz zu den mittels herkömmlichen Kopplungsverfahren hergestellten Konjugaten frei von Modifikationen, die von den verwendeten Linkersubstanzen stammen. Die erfindungsgemäßen Konjugate weisen im Falle von Glykoproteinen, die nur eine oder wenige für die Kopplung geeignete Kohlenhydratgruppen aufweisen, z.B. Transferrin, außerdem den Vorteil auf, daß sie hinsichtlich ihrer Bindungsstelle Glykoprotein/Verbindungsfaktor exakt definiert sind.

Darüberhinaus fungiert die Kohlenhydratgruppe als natürlicher Spacer, der einen Abstand von 32 Atomen zwischen das Glykoprotein und die Nukleinsäure-bindende Substanz setzt. Dieser Abstand könnte ausschlaggebend dafür sein, daß das Glykoprotein dem Zelloberflächenprotein, an das es bindet, in für die Internalisierung geeigneter Weise angeboten wird, indem eine Behinderung durch das Nukleinsäure-bindende Molekül weitgehend ausgeschaltet wird.

Ein weiterer Vorteil der erfindungsgemäßen Konjugate besteht darin, daß sie in höherer Ausbeute erhältlich sind als die nach konventionellen Verfahren über Linkersubstanzen hergestellten.

Die Wahl des Glykoproteins wird vor allem durch die Zielzellen bestimmt, z.B. durch bestimmte Oberflächenantigene oder Rezeptoren, die für einen Zelltyp spezifisch bzw. weitgehend spezifisch sind und somit einen gerichteten Import der Nukleinsäure in diesen Zelltyp ermöglichen.

Im Rahmen der vorliegenden Erfindung sind Glykoproteine oder Fragmente davon geeignet, die an die Zelle binden, wodurch die Konjugat/DNA-Komplexe, insbesondere über Endozytose, internalisiert werden, oder Glykoprotein (fragmente), deren Bindung/Internalisierung über Fusion mit Zellmembranelementen erfolgt.

Wesentlich für die Eignung von Glykoprotein(fragmenten) im Rahmen der vorliegenden Erfindung ist,
a) daß sie vom spezifischen Zelltyp, in den die Nukleinsäure eingebracht werden soll, erkannt werden und ihre Bindungsfähigkeit nicht oder nicht wesentlich beeinträchtigt wird, wenn sie über eine oder mehrere ihrer Kohlenhydratketten mit dem Verbindungsfaktor konjugiert werden, und
b) daß sie im Rahmen dieser Eigenschaft in der Lage sind, Nukleinsäure auf dem von ihnen benutzten Weg in die Zelle "huckepack" mitzunehmen.

Unter der Voraussetzung, daß sie die unter a) und b) definierten Bedingungen erfüllen, sind grundsätzlich alle Glykoproteine für die Anwendung im Rahmen der vorliegenden Erfindung geeignet, die innerhalb ihres Kohlenhydratanteils eine Gruppierung aufweisen, die direkt oder nach Aktivierung zur Reaktion mit dem Verbindungsfaktor fähig sind. Dazu zählen vor allem Glykoproteine mit Kohlenhydratgruppen, bei denen durch Oxidation eine oder mehrere Aldehydgruppen entstehen (z.B. im Fall von Galaktose durch enzymatische Oxidation mit Galaktoseoxidase (Morell et al., 1966)).

Besonders geeignet sind Glykoproteine mit Kohlenhydratketten, die terminale Sialinsäuren (N-Acetylneuraminsäuren, NeuNAc) enthalten. Im Rahmen der vorliegenden Erfindung wurde festgestellt, daß sich Conalbumin (Transferrin aus Hühnereiern), das keine Sialinsäuren in den Kohlenhydratgruppen aufweist, im Gegensatz zu Humantransferrin nur in geringem Ausmaß (weniger als 5 %) über seinen Kohlenhydratanteil mit Polylysin konjugieren läßt.

Als Glykoproteine kommen - neben den bereits erwähnten Transferrin und gp120 - für die Verwendung im Rahmen der vorliegenden Erfindung Virusglykoproteine in Betracht, die am Eintritt des Virus in die Zelle, entweder über Fusion mit der Zellmembran oder über Endozytose, beteiligt sind. Dazu zählen die Herpes simplex Virus-Glykoproteine gB und gD, das Vesicular Stomatitis Virus-Glykoprotein und das Influenza Virus-Glykoprotein: von diesen Glykoproteinen ist bekannt, daß sie beim Eintritt des Virus in die Zelle eine wesentliche Rolle spielen (Butcher et al.,1990; Lisanti et al. 1990; Whitt et al., 1990); vom Influenza-Glykoprotein wurde außerdem gezeigt, daß es für den Transport von mit DNA beladenen Liposomen in die Zelle herangezogen werden kann (Lapidot und Loyter, 1990). Zu geeigneten Glykoproteinen zählen außerdem glykosylierte Zytokine, wie Interleukin-1 und Interleukin 2.

Eine Gruppe von Glykoproteinen, die im Rahmen der vorliegenden Erfindung breiten Einsatz finden können, sind, insbesondere monoklonale, Antikörper gegen ein Zelloberflächenprotein der Zielzelle, in die die Nukleinsäure importiert werden soll, die in der konstanten Region der schweren Kette geeignete Kohlenhydratseitenketten, insbesondere solchen mit endständigen Sialinsäuren aufweisen.

Glykoprotein-Liganden bzw. Antikörper, von denen die Kohlenhydratstruktur bzw. deren Rolle für die Internalisierung unbekannt oder nicht vollständig aufgeklärt ist, können auf ihre Eignung zur Herstellung der erfindungsgemäßen Konjugate untersucht werden, indem in Vorversuchen ihre Kopplungsfähigkeit mit verschiedenen Verbindungsfaktoren geprüft und die gegebenenfalls erhaltenen Konjugate daraufhin untersucht werden, ob sie mit Nukleinsäure Komplexe bilden können, die in die jeweiligen Zielzellen importiert werden.

Voraussetzung für die Eignung von Substanzen als Verbindungsfaktoren im Rahmen der vorliegenden Erfindung ist ihre Fähigkeit zur Reaktion mit einer Gruppierung im, gegebenenfalls modifizierten, Kohlenhydratanteil des Glykoproteins. Dazu zählen Substanzen, die eine oder mehrere Gruppierungen enthalten, die mit der Aldehydfunktion der oxidierten Kohlenhydratgruppe des Glykoproteins reagieren können, insbesondere Substanzen mit einer oder mehreren primären und/oder sekundären Aminogruppen oder mit Hydrazin- bzw. Hydrazidgruppen.

Im Rahmen der vorliegenden Erfindung bevorzugte Verbindungsfaktoren sind Substanzen, die aufgrund ihres polykationischen Charakters an Nukleinsäure binden können. Zu geeigneten organischen Polykationen zählen homologe Polypeptide wie Polylysin, Polyornithin oder heterologe Polykationen mit zwei oder mehr unterschiedlichen Aminosäuren, wobei diese Polykationen verschiedene Kettenlänge aufweisen können, weiters nichtpeptidische synthetische Polykationen wie Polyethylenimin. Geeignet sind weiters natürliche DNA bindende Proteine polykationischen Charakters wie Histone oder Protamine bzw. Analoge oder Fragmente davon.

Die Größe der Polykationen wird bevorzugt so gewählt, daß die Summe der positiven Ladungen etwa 20 bis 500 beträgt, sie wird auf die zu transportierende Nukleinsäure abgestimmt.

Neben Polykationen, die aufgrund von Ladungswechselwirkungen an Nukleinsäure binden, können als Verbindungsfaktoren auch Substanzen verwendet werden, die aufgrund anderer Mechanismen eine Verbindung zwischen dem Glykoprotein DNA herstellen, z.B. aufgrund interkalierender Wirkung. Beispiele für solche Substanzen sind Ethidium- oder Acridin-hältige Verbindungen.

Geeignet sind weiters Verbindungsfaktoren, die durch Einführung einer oder mehrere spezieller Gruppierungen, die für die Kopplung an den Kohlenhydratteil des Glykoproteins geeignet sind, modifiziert wurden. Ein Beispiel dafür ist die Einführung von Hydrazidgruppen in Polyarginin, das erst aufgrund einer solchen Modifikation zur Reaktion mit den Aldehydfunktionen der Kohlenhydratkette des Glykoproteins befähigt ist. Der Vorteil solcher Verbindungsfaktoren besteht darin, daß die Bindungsstelle bezüglich des Verbindungsfaktors exakt definiert werden kann.

Das molare Verhältnis Glykoprotein:Polykation beträgt bevorzugt 10:1 bis 1:10, wobei die maximale Anzahl von Polykationmolekülen pro Glykoprotein-Molekül durch die maximal für die Kopplung verfügbaren Gruppen in den Kohlenhydratketten des Glykoproteins definiert wird.

Ein bevorzugtes Verfahren zur Herstellung der erfindungsgemäßen Glykoprotein-Verbindungsfaktor-Konjugate, das ebenfalls Gegenstand der vorliegenden Erfindung ist, besteht darin, das Glykoprotein unter schonenden Bedingungen zu einer im Kohlenhydratanteil eine oder mehrere Aldehydgruppen enthaltenden Form zu oxidieren und das oxidierte Glykoprotein mit einer Nukleinsäure-bindenden Substanz zu koppeln, die eine oder mehrere Gruppierungen enthält, die befähigt sind, mit der Aldehydfunktion des oxidierten Glykoproteins zu reagieren.

Bevorzugt enthält die Nukleinsäure-bindende Substanz eine oder mehrere primäre und/oder sekundäre Aminogruppen oder Hydrazin- bzw. Hydrazidgruppen. Im Falle der Kopplung mit einer Aminogruppen enthaltenden Substanz wird die Reaktion unter reduzierenden Bedingungen durchgeführt.

Besonders bevorzugt im Rahmen der vorliegenden Erfindung ist ein Verfahren, bei dem eine oder mehrere terminale Sialinsäuren der Kohlenhydratkette(n) des Glykoproteins zur Aldehydform oxidiert werden. Ein bevorzugtes Oxidationsmittel ist Perjodat, insbesondere Natriumperjodat.

Als Substanzen zur Schaffung reduzierender Bedingungen bei der Kopplung der Aldehydform des Glykoproteins mit Aminogruppen enthaltenden Verbindungsfaktoren sind Reduktionsmittel geeignet, die unter für das Glykoprotein schonenden Bedingungen Schiff'sche Basen selektiv reduzieren. Bevorzugt für den Einsatz als Reduktionsmittel im erfindungsgemäßen Verfahren sind Natriumcyanoborhydrid oder tertiäres Butylaminboran.

Das Verfahren wird bevorzugt bei niedrigen Temperaturen, insbesondere 0°C bis Raumtemperatur, durchgeführt.

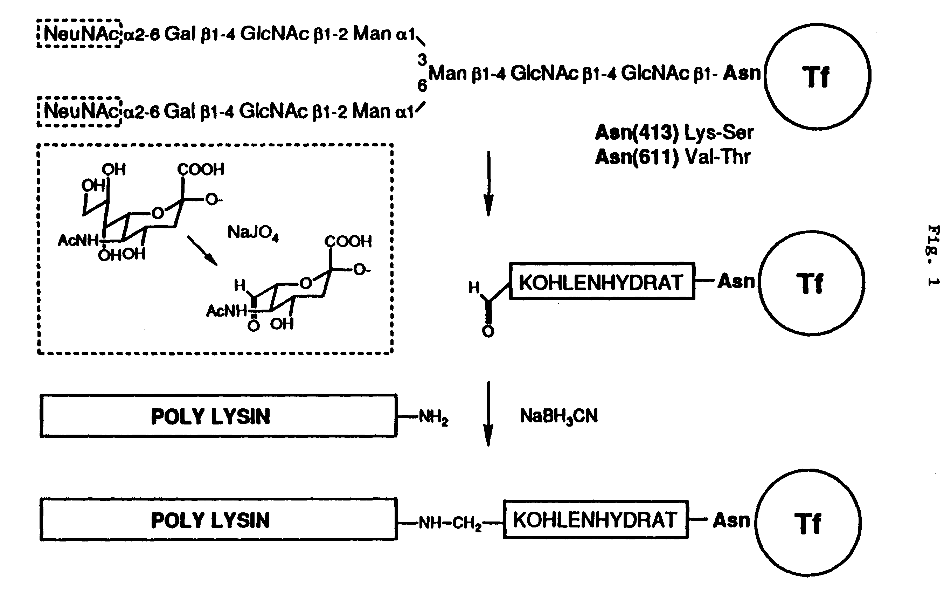

Das bevorzugte Verfahren ist in Fig. 1 anhand von Transferrin-Polylysin-Konjugaten schematisch dargestellt.

Eine weitere Ausgestaltung des erfindungsgemäßen Verfahrens besteht darin, endständige Galaktosereste mit Galaktoseoxidase zu behandeln und die erhaltene oxidierte Form des Glykoproteins mit dem Verbindungsfaktor zu koppeln. Gegebenenfalls können vor Oxidation der Galaktosereste endständige Sialinsäuren entfernt werden, wie von Morell et al., 1966, beschrieben.

Die Mechanismen der einzelnen Reaktionsstufen sind dem Durchschnittsfachmann geläufig; es liegt daher im Rahmen seiner Fähigkeiten, die Bedingungen für die einzelnen Verfahrensschritte den jeweiligen individuellen Bedürfnissen anzupassen.

Im Falle von Antikörper-Polykation-Konjugaten (bzw. Antikörper-Konjugaten mit anderen, Aminogruppen aufweisenden Verbindungsfaktoren) kann im allgemeinen nach den Verfahrensprinzipien vorgegangen werden, die für die Kopplung von Antikörpern an NH₂-Gruppen enthaltende Säulenmatrices verwendet werden.

Gegenstand der Erfindung sind weitere Glykoprotein-Verbindungsfaktor/Nukleinsäure-Komplexe, in denen die erfindungsgemäßen Konjugate mit einer in die Zielzellen zu transportierenden Nukleinsäure komplexiert sind.

Die erfindungsgemäßen Komplexe werden effizient in Zellen, in die das native Glykoprotein internalisiert wird, aufgenommen und die importierte DNA exprimiert, wobei mit steigendem Konjugatgehalt eine erhöhte Expression der DNA in der Zelle beobachtet wurde.

Bei den in die Zelle zu transportierenden Nukleinsäuren kann es sich um DNAs oder RNAs handeln, wobei hinsichtlich der Nukleotidsequenz keine Beschränkungen bestehen. Die Nukleinsäuren können modifiziert sein, sofern (im Fall von Polykationen als Verbindungsfaktor) die Modifikation den polyanionischen Charakter der Nukleinsäuren nicht beeinträchtigt; zu diesen Modifikationen zählen z.B. die Substitution der Phosphodiestergruppe durch Phosphorothioate oder in der Verwendung von Nukleosidanalogen. Derartige Modifikationen sind dem Fachmann geläufig, eine Übersicht über Vertreter modifizierter Nukleinsäuren, die im allgemeinen als Nukleinsäureanaloga bezeichnet werden, sowie deren Wirkprinzip, sind in der Übersicht von Zon (1988) angegeben.

Bezüglich der Größe der Nukleinsäuren ermöglicht die Erfindung ebenfalls Anwendungen in einem weiten Bereich. Hinsichtlich der oberen Grenze besteht keine durch die erfindungsgemäßen Konjugate bedingte prinzipielle Limitierung, solange gewährleistet ist, daß die Glykoprotein-Verbindungsfaktor/Nukleinsäure-Komplexe in die Zelle befördert werden. Eine etwaige Begrenzung nach unten ergibt sich aus anwendungsspezifischen Gründen, weil z.B. Antisense-Oligonukleotide unter etwa 10 Nukleotiden auf Grund zu geringer Spezifität kaum für die Anwendung in Frage kommen. Mit Hilfe der erfindungsgemäßen Konjugate können auch Plasmide in die Zelle befördert werden.

Es ist auch möglich, gleichzeitig verschiedene Nukleinsäuren mit Hilfe der erfindungsgemäßen Konjugate in die Zelle zu befördern.

Beispiele für geeignete Nukleinsäuren sind die bereits angeführten Antisenseoligonukleotide oder Ribozyme mit Komplementarität zu für die Virusreplikation essentiellen Genabschnitten.

Bevorzugt als Nukleinsäureanteil der erfindungsgemäßen Glykoprotein-Polykation-Nukleinsäurekomplexe mit inhibierender Wirkung aufgrund von Komplementarität ist Antisense-DNA, Antisense-RNA oder ein Ribozym bzw. das dafür kodierende Gen. Bei der Anwendung von Ribozymen und Antisense-RNAs ist es besonders vorteilhaft, die dafür kodierenden Gene, gegebenenfalls zusammen mit einem Carriergen, einzusetzen. Durch die Einführung des Gens in die Zelle wird gegenüber dem Import der RNA als solcher eine beträchtliche Amplifikation der RNA und damit ein für die angestrebte Hemmung der biologischen Reaktion ausreichender Vorrat gewährleistet. Besonders geeignete Carriergene sind die für die Transkription durch Polymerase III erforderlichen Transkriptionseinheiten, z.B. tRNA-Gene. In diese können z.B. Ribozymgene derart eingefügt werden, daß bei Transkription das Ribozym Teil eines kompakten Polymerase III-Transkripts ist. Geeignete genetische Einheiten, enthaltend ein Ribozymgen und ein von Polymerase III transkribiertes Carriergen, sind in der Europäischen Patentanmeldung A1 0 387 775 geoffenbart. Mit Hilfe des Transportsystems gemäß der vorliegenden Erfindung kann die Wirkung dieser genetischen Einheiten verstärkt werden, indem eine erhöhte Ausgangskonzentration des Gens in der Zelle gewährleistet wird.

Als Zielsequenzen für die Konstruktion komplementärer Antisenseoligonukleotide oder Ribozyme bzw. der dafür kodierenden Gene, die bei der Therapie von AIDS zur Anwendung kommen können, sind grundsätzlich sämtliche Sequenzen des HIV-Gens geeignet, deren Blockierung die Inhibierung der viralen Replikation und Expression zur Folge hat. In erster Linie in Frage kommende Zielsequenzen sind Sequenzen mit regulatorischer Funktion, vor allem des tat-, rev- oder nef-Gens. Geeignete Sequenzen sind weiters die Initiations-, Polyadenylierungs-, Splicing tRNA Primer-Bindungsstelle (PBS) der LTR-Sequenz oder die tar-Sequenz.

Außer Nukleinsäuremolekülen, die aufgrund ihrer Komplementarität zu viralen Genen inhibieren, können auch Gene mit anderem Wirkmechanismus eingesetzt werden, z.B. solche, die für Virusproteine, enthaltend sog. trans-dominante Mutationen, kodieren (Herskowitz, 1987). Die Expression der Genprodukte in der Zelle resultiert in Proteinen, die in ihrer Funktion das entsprechende Wildtyp-Virusprotein dominieren, womit dieses seine für die Virusreplikation normalerweise geleistete Aufgabe nicht erfüllen kann und die Virusreplikation wirksam inhibiert wird. Geeignet sind grundsätzlich trans-dominante Mutanten von Virusproteinen, die für die Replikation und Expression erforderlich sind, z.B. Gag-, Tat- und Rev-Mutanten, von denen inhibierende Wirkung auf die HIV-Replikation nachgewiesen wurde (Trono et al., 1989; Green et al., 1989; Malim et al., 1989).

Vertreter therapeutisch wirksamer Nukleinsäuren sind weiters solche mit inhibierender Wirkung auf Onkogene.

Mit Hilfe der vorliegenden Erfindung können auch Gene bzw. Abschnitte davon in die Zelle transportiert werden, deren Expressionsprodukte eine Funktion bei der Signalübertragung haben, um auf diese Weise die Signalübertragung in den Zielzellen, z.B. CD4+-Zellen, insbesondere T-Zellen, positiv zu beeinflussen und damit z.B. die Überlebensfähigkeit von T-Zellen zu steigern.

Prinzipiell sind sämtliche Gene bzw. Genabschnitte im Rahmen der vorliegenden Erfindung geeignet, die in den das Zelloberflächenprotein exprimierenden Zellen einen therapeutischen oder gentherapeutischen Effekt haben.

Beispiele für Nukleinsäuren, die im Zuge der humanen Gentherapie verwendet und mit Hilfe der vorliegenden Erfindung in die Zelle eingeführt werden können, sind die DNAs, kodierend für Faktor VIII (Wood et al., 1984), Faktor IX (Hämophilie; Kurachi et al., 1982), Adenosindeaminase (Servere Combined Immune Deficiency, SCID; Valerio et al., 1984), α-1-Antitrypsin (Lungenemphysem; Ciliberto et al., 1985) oder das "Cystic Fibrosis Conductance Regulator Gene" (Riordan et al., 1989).

Das Verhältnis Nukleinsäure:Konjugat kann innerhalb eines weiten Bereichs schwanken, wobei es nicht unbedingt erforderlich sein muß, sämtliche Ladungen der Nukleinsäure zu neutralisieren. Dieses Verhältnis wird von Fall zu Fall nach Kriterien wie Größe und Struktur der zu transportierenden Nukleinsäure, Größe des Polykations, Anzahl und Verteilung seiner Ladungen, derart einzustellen sein, daß ein für die jeweilige Anwendung günstiges Verhältnis zwischen Transportfähigkeit und biologischer Aktivität der Nukleinsäure besteht. Dieses Verhältnis kann zunächst grob eingestellt werden, etwa an Hand der Verzögerung der Wanderungsgeschwindigkeit der DNA in einem Gel (z.B. mittels "Mobility Shift" auf einem Agarosegel) oder durch Dichtegradientenzentrifugation. Nach Erhalt dieses vorläufigen Verhältnisses kann es zweckmäßig sein, im Hinblick auf die in der Zelle maximal verfügbare Aktivität der Nukleinsäure Transportversuche mit dem radioaktiv markierten Komplex durchzuführen und den Konjugat-Anteil gegebenenfalls derart zu reduzieren, daß die verbleibenden negativen Ladungen der Nukleinsäure dem Transport in die Zelle nicht hinderlich sind.

Die Herstellung der Glykoprotein-Polykation/Nukleinsäure-Komplexe kann nach in an sich für die Komplexierung polyionischer Verbindungen bekannten Methoden vorgenommen wurden. Eine Möglichkeit, unkontrollierte Aggregation bzw. Ausfällung zu vermeiden, besteht darin, die beiden Komponenten bei hoher Verdünnung (≤50 µg/ml) zu mischen.

Im Rahmen der vorliegenden Erfindung wurde als DNA-Komponente das Luciferase-Gen als Reporter-Gen verwendet. In Vorversuchen mit Transferrin-Polykation/DNA-Komplexen, in denen das Luciferase-Gen als Reportergen verwendet wurde, wurde festgestellt, daß aus der Effizienz des Imports des Luciferase-Gens auf die Anwendbarkeit anderer Nukleinsäuren geschlossen werden kann und die verwendete Nukleinsäure in qualitativer Hinsicht kein limitierender Faktor für die Anwendung von Protein-Polykation-DNA-Komplexen ist.

Die in höhere eukaryotische Zellen aufnehmbaren Glykoprotein-Verbindungsfaktor-Nukleinsäure-Komplexe können zusätzlich eine oder mehrere Polykationen in nicht-kovalent gebundener Form, die gegebenenfalls identisch sind mit dem Polykation im Konjugat, derart enthalten, daß die durch das Konjugat erzielte Internalisierung und/oder Expression der Nukleinsäure gesteigert wird.

Mit Hilfe derartiger Maßnahmen (Wagner et al., 1991), die Gegenstand der WO 92/13570 sind, wird bei zumindest gleicher Transfektions/Expressions-Effizienz eine geringere Menge, bezogen auf die in die Zelle zu importierende Nukleinsäure-Menge, Glykoprotein-Polykation-Konjugat benötigt, was einerseits einen geringeren Synthese-Aufwand bedeutet. Eine geringere Konjugatmenge kann andererseits dann von Vorteil sein, wenn der Effekt vermieden werden soll, daß durch eine größere Anzahl von Glykoprotein-Molekülen innerhalb eines Komplexes gleich mehrere benachbarte "Andock-Stellen" besetzt werden und in der Folge für weitere Komplexe nicht mehr verfügbar sein können. Die Anzahl der in den Komplexen enthaltenen Menge an Glykoprotein auf das erforderliche Minimum zu beschränken, d.h. die Konjugatmenge möglichst gering zu halten und mit freiem Polykation zu verdünnen, ist insbesondere auch dann von Vorteil, wenn die Zahl der Zelloberflächenproteine, an die der Komplex bindet, beim jeweiligen Zelltyp gering ist.

Mit Hilfe solcher Maßnahmen kann die Leistungsfähigkeit von Konjugaten, die für sich weniger effizient sind, beträchtlich erhöht werden und die Leistungsfähigkeit von an sich schon sehr effizienten Konjugaten noch weiter gesteigert werden.

Hinsichtlich der qualitativen Zusammensetzung der erfindungsgemäßen Komplexe werden im allgemeinen zunächst die in die Zelle zu importierende Nukleinsäure und das Glykoprotein festgelegt. Die Nukleinsäure wird vor allem durch den in der Zelle zu erzielenden biologischen Effekt definiert, z.B. durch die Zielsequenz des zu inhibierenden oder - im Falle der Anwendung im Rahmen der Gentherapie - des zur Expression zu bringenden Gens bzw. Genabschnitts, z.B. zwecks Substitution oder Addition eines defekten Gens. Gegebenenfalls ist die Nukleinsäure modifiziert, bedingt z.B. durch eine für den jeweiligen Anwendungsfall erforderliche Stabilität.

Ausgehend von der Festlegung von Nukleinsäure und Glykoprotein wird das Polykation auf diese Parameter abgestimmt, wobei die Größe der Nukleinsäure, vor allem im Hinblick auf eine weitgehende Neutralisierung der negativen Ladungen, eine wesentliche Rolle spielt.

Für die Wahl der gegebenenfalls in den Komplexen enthaltenen nicht-kovalent gebundenen Polykationen ist entscheidend, daß durch ihren Zusatz gegenüber der durch die Konjugate erzielbaren Internalisierung/Expression der Nukleinsäure eine Steigerung bewirkt wird.

Ebenso wie die qualitative Zusammensetzung richtet sich auch die quantitative Zusammensetzung der Komplexe nach mehreren Kriterien, die miteinander in funktionellem Zusammenhang stehen, z.B. ob und in welchem Ausmaß es erforderlich oder erwünscht ist, die Nukleinsäure zu kondensieren, welche Ladung der Gesamtkomplex aufweisen soll, in welchem Ausmaß Bindungs- und Internalisierungsfähigkeit für den jeweiligen Zelltyp gegeben ist und in welchem Ausmaß deren Steigerung erwünscht bzw. erforderlich ist. Weitere Parameter für die Zusammensetzung des Komplexes sind die Zugänglichkeit des Glykoproteins für das Zelloberflächenprotein, wobei dafür die Art maßgeblich ist, wie das Glykoprotein innerhalb des Komplexes der Zelle gegenüber präsentiert wird. Wesentlich ist weiters die Zugänglichkeit der Nukleinsäure in der Zelle, um deren bestimmungsgemäße Funktion auszuüben.

Die in nicht kovalent-gebundener Form in den Komplexen gegebenenfalls enthaltenen Polykationen können gleich oder verschieden sein von den im Konjugat enthaltenen. Ein wesentliches Kriterium für deren Auswahl ist die Größe der Nukleinsäure, insbesondere im Hinblick auf deren Kondensierung; bei kleineren Nukleinsäure-Molekülen ist im allgemeinen eine Kompaktierung nicht erforderlich. Die Auswahl der Polykationen nach Art und Menge wird außerdem auf das Konjugat abgestimmt, wobei vor allem das im Konjugat enthaltene Polykation zu berücksichtigen ist: ist das Polykation z.B eine Substanz, die keine oder nur geringe Fähigkeit zur DNA-Kondensation aufweist, ist es im Hinblick auf eine effiziente Internalisierung der Komplexe im allgemeinen zweckmäßig, solche Polykationen einzusetzen, die diese Eigenschaft in stärker ausgeprägtem Maß besitzen. Ist das im Konjugat enthaltene Polykation selbst eine Nukleinsäure kondensierende Substanz und bewirkt bereits eine im Hinblick auf effiziente Internalisierung ausreichende Kompaktierung der Nukleinsäure, wird zweckmäßigerweise ein Polykation verwendet, das aufgrund anderer Mechanismen eine Steigerung der Expression bewirkt.

Wesentlich für das gegebenenfalls im Komplex zusätzlich enthaltene nicht-kovalent gebundene Polykation ist seine Fähigkeit, Nukleinsäure zu kondensieren und/oder diese vor unerwünschtem Abbau in der Zelle zu schützen.

Gegenstand der Erfindung ist weiters ein Verfahren zum Einführen von Nukleinsäure(n) in menschliche oder tierische Zellen, wobei bevorzugt ein unter physiologischen Bedingungen löslicher Glykoprotein-Verbindungsfaktor/Nukleinsäure-Komplex mit den Zellen in Berührung gebracht wird.

Es kann für bestimmte Ausführungsformen der vorliegenden Erfindung vorteilhaft sein, Bedingungen zu schaffen, unter denen der Abbau der in die Zelle importierten Nukleinsäure verringert oder ausgeschaltet wird.

Bedingungen, unter denen der Abbau von Nukleinsäuren inhibiert wird, können durch Zusatz sog. lysosomatroper Substanzen geschaffen werden. Von diesen Substanzen ist bekannt, daß sie die Aktivität von Proteasen und Nukleasen in Lysosomen hemmen und somit den Abbau von Nukleinsäuren verhindern können (Luthman und Magnusson, 1983).

Zu diesen Substanzen zählen Chloroquin, Monensin, Nigericin, Ammoniumchlorid und Methylamin.

Das Erfordernis für die Anwendung einer Substanz aus der Gruppe der lysosomatropen Substanzen im Rahmen der vorliegenden Erfindung richtet sich u.a. nach dem zu behandelnden Zelltyp oder bei Verwendung unterschiedlicher Glykoproteine gegebenenfalls nach unterschiedlichen Mechanismen, über die die Aufnahme der Komplexe in die Zelle erfolgt. So wurde z.B. im Rahmen der vorliegenden Erfindung eine unterschiedliche Beeinflussung des DNA-Imports in die Zelle durch Chloroquin bei Verwendung unterschiedlicher Antikörper (monoklonale Anti-CD4-Antikörper) festgestellt.

Es ist in jedem Fall notwendig, das Erfordernis bzw. die Eignung derartiger Substanzen im Rahmen der vorliegenden Erfindung in Vorversuchen zu testen.

Die jeweils geeigneten Transfektionsbedingungen werden auf die zu transfizierenden Zellen bzw. deren Zustand abgestimmt. Im Falle des Einsatzes von Transferrin-Konjugaten können dazu Maßnahmen eingesetzt werden, mit denen die Transferrin-Rezeptorzahl erhöht wird.

Geeignete Bedingungen, denen die Zellen ausgesetzt werden, um die Transferrin-Rezeptormenge zu erhöhen, bestehen z.B. darin, die Zelle mit Substanzen in Berührung zu bringen, die geeignet sind, die Hämkonzentration im Inneren der Zelle zu senken.

Dabei handelt es sich bevorzugt um Substanzen, die eine Verringerung der Hämkonzentration in der Zelle bewirken, indem sie
a) die Protoporphyrin IX-Biosynthese hemmen
b) die Hämbildung verhindern
c) den Hämabbau fördern.

Zu den Substanzen der Gruppe a) zählt Succinylaceton, wobei die Erhöhung der Transferrin-Rezeptorzahl wahrscheinlich auf die Inhibierung der Protoporphyrin IX-Biosynthese zurückzuführen ist.

Zu den Substanzen der Gruppe b) zählen diejenigen, die einen Eisenmangel in der Zelle induzieren, z.B. der Eisenchelatbildner Desferrioxamin, der vermutlich eine Erhöhung der Transferrin-Rezeptorzahl bewirkt, weil die Umwandlung von Protoporphyrin IX in Häm verhindert wird. Grundsätzlich sind all die Substanzen, insbesondere Eisenchelatbildner, die in die Zelle aufgenommen werden können und die für Desferrioxamin festgestellte Wirkung auf die für die Hämbildung verfügbare Eisenmenge haben, zur Erhöhung der Transferrin-Rezeptorzahl geeignet.

Der angeführten Substanzgruppe kommt für die Anwendung von Transferrin-Komplexen im Rahmen der vorliegenden Erfindung weiters insofern Bedeutung zu, als sie offensichtlich geeignet sind, die Kompetition von nativem Transferrin mit den Transferrin-Polykation/Nukleinsäure-Komplexen um die Bindung an den Rezeptor aufzuheben. Durch die Vorbehandlung von Zellen - in vivo oder in vitro - mit solchen Substanzen kann das nicht Transferrin-gebundene Eisen entfernt werden, womit kein Eisen mehr für das aus der Zelle ausgeschleuste Transferrin verfügbar ist und dieses somit als Apotransferrin nicht mit den Transferrin-Polykation/Nukleinsäure-Komplexen um die Bindung an den Transferrin-Rezeptor konkurriert. Durch die Entfernung von freiem Eisen kann andererseits eine Senkung der Transferrin-Rezeptorzahl durch Anwesenheit von freiem Eisen verhindert werden.

Zur Gruppe c) zahlen Substanzen, die den Hämabbau in der Zelle induzieren bzw. stimulieren, worauf die Zelle mit Erhöhung der Transferrin-Rezeptor-Konzentration bzw. der Affinität von Transferrin zu seinem Rezeptor bzw. der Rezeptor-Recycling-Rate reagiert. Es wurde festgestellt, daß mit Hilfe dieser Substanzen die Aufnahme von DNA in die Zelle verbessert werden kann, wobei beobachtet wurde, daß Kombinationen von Substanzen der Gruppen a) bis c) einen additiven Effekt, bestimmt über die Aktivität des exprimierten Reportergens, aufwiesen.

Gegenstand der Erfindung sind weiters pharmazeutische Zubereitungen, enthaltend als wirksame Komponente eine oder mehrere therapeutisch oder gentherapeutisch wirksame Nukleinsäure(n), komplexiert mit einem Glykoprotein-Verbindungsfaktor-Konjugat (Glykoprotein-Polykation-Konjugat und Nukleinsäure können auch getrennt vorliegen und unmittelbar vor der therapeutischen Anwendung komplexiert werden).

Als therapeutisch wirksame Nukleinsäuren kommen die bereits angeführten Antisenseoligonukleotide oder Ribozyme bzw. die dafür kodierenden Gene oder für trans-dominante Mutanten kodierende Gene in Betracht, die inhibierende Wirkung auf in den jeweiligen Zielzellen enthaltene endogene oder exogene Gene oder Genprodukte haben. Darunter sind z.B. diejenigen Gene zu verstehen, die aufgrund ihrer Sequenzspezifität (Komplementarität zu Zielsequenzen, Kodierung für trans-dominante Mutanten (Herskowitz, 1987)) eine intrazelluläre Immunität (Baltimore, 1988) gegen HIV bewirken und bei der Therapie des AIDS-Syndroms oder zur Verhinderung der Aktivierung des Virus nach der Infektion verwendet werden können.

In der pharmazeutischen Zubereitung können übliche pharmazeutisch annehmbare Träger, wie Kochsalz oder phosphatgepuffertes Kochsalz oder jeder Träger, in dem die erfindungsgemäßen Komplexe die für die Anwendung geeignete Löslichkeit aufweisen, enthalten sein. Vorschriften für die Formulierung pharmazeutischer Zubereitungen sind einschlägigen Handbüchern entnehmbar (z.B. Lehrbuch der pharmazeutischen Technologie, 1984; Pharmazeutische Technologie, 1978).

Die pharmazeutischen Zubereitungen können verwendet werden, um im menschlichen oder tierischen Organismus virale Sequenzen, z.B. HIV oder verwandte Retroviren zu inhibieren. Ein Beispiel für die therapeutische Anwendung durch Inhibierung eines verwandten Retrovirus ist die Behandlung der proliferativen T-Zelleukämie, die durch das HTLV-1 Virus hervorgerufen wird.

Neben der Behandlung viraler T-Zelleukämien kommt für die Anwendung der vorliegenden Erfindung auch die Therapie nicht-viraler Leukämien in Betracht. Die Beteiligung von Onkogenen (abl, bcr, ras, rat, c-myc, N-myc) an der Entstehung lymphatischer Leukämien wurde in jüngster Zeit nachgewiesen; die Existenz weiterer Onkogene wird aufgrund von beobachteten spezifischen Chromosomentranslokationen für wahrscheinlich erachtet. Die Klonierung dieser Onkogene bietet die Grundlage für die Konstruktion onkogen-inhibierender Nukleinsäuremoleküle und damit für eine weitere therapeutische Einsatzmöglichkeit der vorliegenden Erfindung.

Ein weiteres wichtiges Anwendungsgebiet ist die Gentherapie. Im Rahmen der Gentherapie können mit Hilfe der vorliegenden Erfindung all diejenigen Gene bzw. Abschnitte davon in die Zielzellen eingeführt werden, deren Expression in diesem Zelltyp einen therapeutischen Effekt erzielt, z.B. durch Substitution genetisch bedingter Defekte oder Auslösen einer Immunantwort.

Für die therapeutische Anwendung können die erfindungsgemäßen Komplexe als Bestandteil der pharmazeutischen Zubereitung systemisch, z.B. über die intravenöse Route, verabreicht werden. Zielorgane für diese Anwendung sind z.B. die Leber, die Milz, die Lunge, das Knochenmark oder Tumore. Die pharmazeutischen Zubereitungen können auch lokal angewendet werden, z.B. durch direkte Injektion ins Muskelgewebe oder in einen Tumor oder durch lokale Verabreichung im Gastrointestinaltrakt.

Die therapeutische Anwendung kann auch ex vivo erfolgen, wobei die behandelten Zellen, z.B. Knochenmarkszellen oder Hepatozyten, in den Körper rückgeführt werden (z.B. Ponder et al., 1991).

### Figurenübersicht

- Fig. 1:: Reaktionsschema der Konjugation von Transferrin mit Polylysin über die Kohlenhydratkette
- Fig. 2:: Isolierung von Transferrin-Polylysin-Konjugaten durch Kationenaustauschchromatographie
- Fig. 3:: Transfektion eukaryotischer Zellen mit Transferrin-Polylysin/DNA-Komplexen
- Fig. 4:: Kompetitive Inhibierung der Transfektion mit Transferrin-Polykation-Konjugaten durch eisenhältiges Transferrin
- Fig. 5:: Transfektion von CD4⁺-Zellen mit gp120-Polylysin/DNA-Komplexen

Die Erfindung wird anhand der nachfolgenden Beispiele illustriert:

### Beispiel 1

### Herstellung von Transferrin-Polylysin-Konjugaten

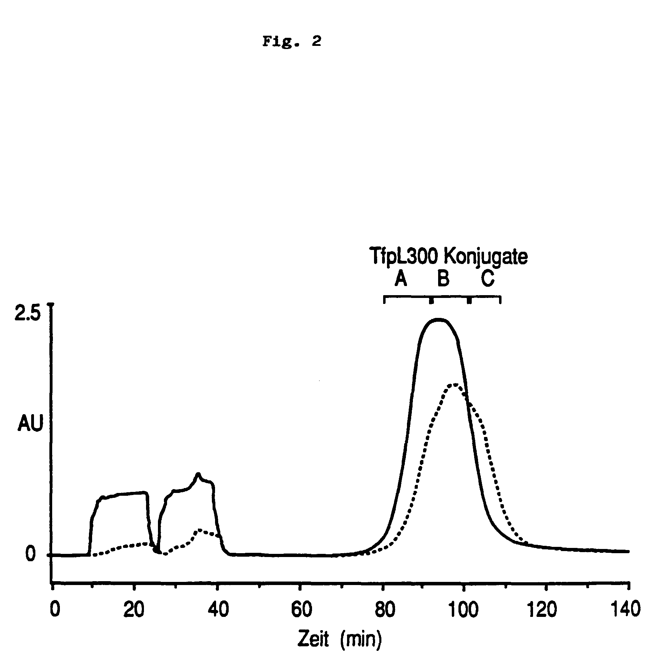

Eine Lösung von 102 mg (1.28 µmol) humanem Transferrin (eisenfrei, Sigma) in 3 ml 30 mM Natriumacetatpuffer, pH 5, wurde einer Gelfiltration auf einer Sephadex G-25 Säule (Pharmacia) unterworfen. Die resultierenden 3.8 ml Lösung wurden auf 0°C gekühlt und 80 µl 30 mM Natriumacetatpuffer pH 5 enthaltend 4 mg (19 µmol) Natriumperjodat hinzugefügt. Die Mischung wurde im Eisbad 90 min lang im Dunklen stehen gelassen. Zur Entfernung der niedermolekularen Produkte wurde eine weitere Gelfiltration durchgeführt (siehe oben), die eine Lösung mit einem Gehalt von ca. 82 mg (1.03 µmol) oxidiertem Transferrin (Messung durch UV-Absorption bei 280 nm und Ninhydrinassay) ergab. (Um die oxidierte Form nachzuweisen, die Aldehyde enthält und bei Färbung mit Anisaldehyd eine Farbreaktion gibt, wurden die Proben auf eine Silikagel-Dünnschichtplatte getropft, getrocknet und die Platten in p-Anisaldehyd/Schwefelsäure/Ethanol (1/1/18) getaucht, getrocknet und erhitzt.) Die modifizierte Transferrin-Lösung wurde rasch (innerhalb 10 bis 15 min) einer Lösung, enthaltend 0.50 µmol fluoreszenzmarkiertes Poly(L)Lysin mit einer durchschnittlichen Kettenlänge von 300 Lysinmonomeren (erhalten durch Reaktion von 34 mg Hydrobromid-Salz während 3 h mit 130 µg Fluoresceinisothiocyanat in Natriumbicarbonatpuffer pH 9 und anschließender Gelfiltration) in 4.5 ml 100 mM Natriumacetat pH 5 unter heftigem Rühren hinzugefügt. Der pH-Wert der Lösung wurde nach 20 min durch Zusatz von 1 M Natriumbicarbonatpuffer auf pH 7.5 eingestellt. Zu der Mischung wurden in Abständen von je 1 h 4 Portionen von je 9.5 mg (150 µmol) Natriumcyanoborhydrid hinzugefügt. Nach 18 h wurden 1.9 ml 5 M Natriumchlorid zugefügt, um die Lösung auf eine Gesamtkonzentration von ca. 0.75 M zu bringen. Die Reaktionsmischung wurde auf eine Kationenaustauschsäule (Pharmacia Mono S HR 10/10) aufgebracht und mit einem Salzgradienten von 0.75 M bis 2.5 M Natriumchlorid mit einem konstanten Gehalt von 25 mM HEPES pH 7.3 fraktioniert. Die hohe Salzkonzentration beim Laden der Säule und ab Beginn des Gradienten war wesentlich für die Gewinnung der Polykation-Konjugate. Etwas Transferrin (ca. 30 %) zusammen mit einer schwachen Fluoreszenzaktivität eluierte im Durchfluß; die Hauptmenge von fluoreszenzmarkiertem Konjugat eluierte bei einer Salzkonzentration zwischen 1.35 M und 1.9 M (s. Fig. 2: --- UV-Absorption bei 280 nm; .... Fluoreszenz bei 520 nm) und wurde in 3 Fraktionen gepoolt. Diese Fraktionen ergaben (in der Reihenfolge ihrer Elution) nach 2maliger Dialyse gegen 2 l 25 mM HEPES pH 7.3 eine Fraktion A (TfpL300A) mit einem Gehalt an 19 mg (0.24 µmol) Transferrin, modifiziert mit 80 nmol Polylysin, eine Fraktion B (TfpL300B) mit einem Gehalt von 27 mg (0.34 µmol) Transferrin, modifiziert mit 150 nmol Polylysin und eine Fraktion C (TfpL300C), enthaltend 5 mg (62 nmol) Transferrin, modifiziert mit 80 nmol Polylysin. Die Gesamtausbeute betrug 50 %, bezogen auf Transferrin, und 62 %, bezogen auf Polylysin. Die Transferrin-Konjugate wurden, sofern sie nicht sofort verwendet wurden, nach Schockgefrieren in flüssigem Stickstoff bei -20°C in eisenfreier Form gelagert. Vor dem Einbau von Eisen wurden Proben (0.5 bis 1 mg) mit Natriumchlorid auf eine physiologische Salzkonzentration (150 mM) gebracht. Der Eiseneinbau wurde durch Hinzufügen von 6 µl 10 mM Eisen(III)citratpuffer (enthaltend 200 mM Citrat, durch Zugabe von Natriumbicarbonat auf einen pH-Wert von 7.8 eingestellt) pro mg Transferrin-Gehalt vorgenommen. Die eisenhältigen Konjugate wurden vor ihrer Verwendung für die DNA-Komplexbildung in kleine Aliquots aufgeteilt, schockgefroren und bei -20°C in flüssigem Stickstoff oder Trockeneis/Ethanol aufbewahrt (Diese Maßnahme erwies sich als zweckmäßig, nachdem sich gezeigt hatte, daß mehrmaliges Auftauen und Einfrieren den Verderb der Konjugate zur Folge hat).

### Beispiel 2

### Transfektion eukaryotischer Zellen mit Transferrin-Polylysin/DNA-Komplexen

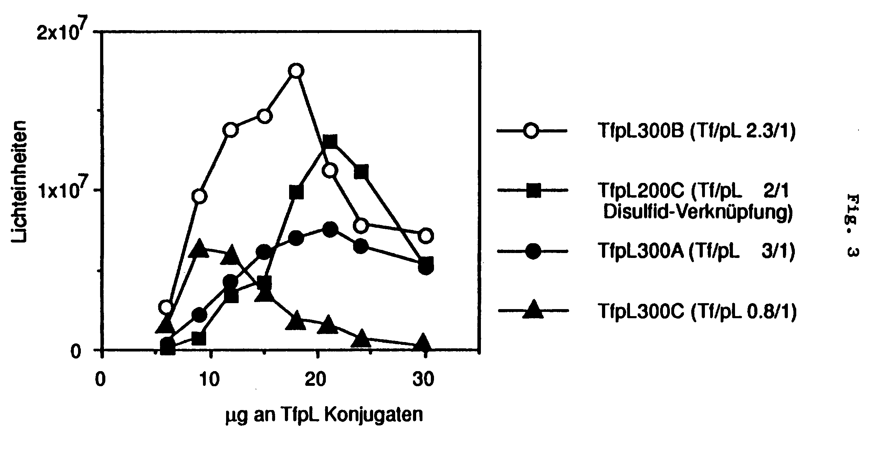

Zellen der Erythroleukämiezellinie K562 wurden in Suspension in RPMI 1640-Medium plus 10 % FCS, 100 Einheiten/ml Penicilin, 100 µg/ml Streptomycin und 2 mM Glutamin bis zum Erreichen einer Zelldichte von 500.000 Zellen/ml gezüchtet, anschließend zweimal mit frischem Medium gewaschen. 20 Stunden vor der Transfektion wurden die Zellen zu ca. 200.000 Zellen/ml in frisches Medium, enthaltend 50 µM Desferrioxamin (Sigma), gegeben. Vor der Transfektion wurden die Zellen gesammelt, in frischem Medium, enthaltend 10 % FCS (plus 50 µM Desferrioxamin) zu 250.000 Zellen/ml wieder aufgenommen und in eine 24-Napf-Kulturschale gegeben (2 ml/Napf). (Die Vorbehandlung mit Desferrioxamin dient dazu, die Transferrin-Rezeptorzahl um das ca. 5fache zu erhöhen und damit eine Verbesserung der DNA-Aufnahme zu erzielen.)
6µg des DNA-Plasmids pRSVL (enthaltend das Photinus pyralis Luciferasegen unter der Kontrolle des Rous Sarcoma Virus LTR Enhancer/Promoters (Uchida et al., 1977; De Wet et al., 1987), hergestellt mittels Triton-X Lyse Standard-Methode (Maniatis), gefolgt von CsCl/EtBr Gleichgewichtsdichtegradienten-Zentrifugation, Entfärbung mit Butanol-1 und Dialyse gegen 10 mM Tris/HCl pH 7.5, 1 mM EDTA) in 330 µl HBS (150 mM NaCl, 20 mM HEPES pH 7.3) wurden mit den in Fig. 3 angegebenen verschiedenen Mengen von Transferrin-Polylysin-Konjugat in 170 µl HBS gemischt. Nach ca. 30 min bei Raumtemperatur wurden die 500 µl Mischung zu den K652-Zellen hinzugefügt (5 bis 10 min vor Zugabe des DNA/Konjugat-Komplexes wurde die 2 ml Zellprobe mit Chloroquin (Endkonzentration 100 µM) behandelt, um den Abbau der DNA innerhalb der Zelle zu inhibieren). Nach 4stündiger Inkubation bei 37°C wurden die Zellen in vorgewärmtes frisches Medium (ohne Chloroquinzusatz) gewaschen und bei 37°C inkubiert. 20 h nach der Transfektion wurden die Zellen geerntet und Extrakte hergestellt. Zellextraktaliquots, standardisiert auf Proteingehalt, wurden auf Luciferase-Aktivität untersucht, wie in der EP-A1 0388 578 beschrieben. Die Ergebnisse dieser Untersuchungen sind in Fig. 3 wiedergegeben (Zum Vergleich wurden Transferrin-Polylysin-Konjugate, hergestellt mittels herkömmlicher Synthese durch Kopplung über Disulfidbrücken, herangezogen. Die verwendeten TfpL200C-Konjugate hatten sich in Vorversuchen als am effizientesten gezeigt).

### Beispiel 3

### Aufnahme der Transferrin-Polylysin/DNA-Komplexe über den Transferrin-Rezeptor

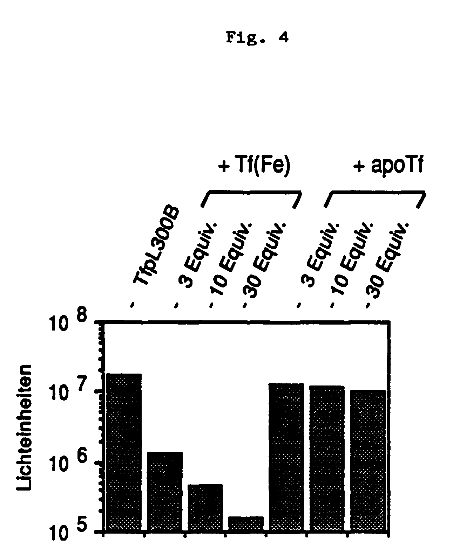

Um festzustellen, ob die Komplexe zwischen DNA und den erfindungsgemäßen Konjugaten über den Transferrin-Rezeptor erfolgt, wurden Komplexe von 6 µg pRSVL mit 18 µg der in Beispiel 1 erhaltenen TfpL300-Konjugate (Fraktion B) zur Transfektion von K562-Zellen herangezogen, wobei die Zellen 30 Minuten vor der Transfektion mit steigenden Mengen von eisenhältigem Transferrin oder eisenfreiem Apotransferrin vorbehandelt wurden. Um mögliche Eisenquellen auszuschalten, wurde in diesen Versuchen serumfreies Medium verwendet. Das Ergebnis dieser Versuche ist in Fig. 4 dargestellt; die angegebenen Äquivalente entsprechen dem Transferrin-Gehalt in den DNA-Komplexen; die Luziferase-Aktivität bezieht sich auf 500.000 Zellen.

### Beispiel 4

### Herstellung von gp120-Polylysin-Konjugaten

Eine Lösung von 3.6 mg (30 nmol) rekombinantem gp120 (Lasky et al., 1986, 1987) in 800 µl 150 mM Natriumacetatpuffer (pH 5) wurde auf 0°C gekühlt und 50 µl 30 mM Natiumacetatpuffer, enthaltend 100 µg (0.46 µmol) Natriumperjodat hinzugefügt. Die Mischung wurde im Dunklen eine Stunde lang im Eisbad stehengelassen. Um die niedermolekularen Produkte zu entfernen, wurde die Reaktionsmischung einer Gelfiltration (Sephadex G-25, 30 mM Natriumacetatpuffer pH 5) unterworfen. Es wurden 1.4 ml Lösung, enthaltend ca. 3.5 mg (29 nmol) oxidiertes gp120 erhalten (Bestimmung mittels UV-Absorption bei 280 nm und Ninhydrin-Assay; die oxidierte Form gibt im Gegensatz zum nicht-modifizierten Protein mit Anisaldehyd-Reagens eine Farbreaktion; vgl. Beispiel 1). Die modifizierte gp120-Lösung wurde einer Lösung mit einem Gehalt von 47 nmol Poly(L)lysin einer durchschnittlichen Kettenlänge von 300 Lysinmonomeren in 60 µl 100 mM Natriumacetat (pH 5) bei Raumtemperatur unter starkem Rühren beigegeben. Der pH-Wert der Lösung wurde nach einer Stunde durch Zufügen von 80 µl 1 M HEPES auf 7.3 eingestellt. Danach wurden in einstündigen Intervallen 3 Portionen von je 0.5 mg (8 µmol) Natriumcyanoborhydrid zugegeben und die Mischung über Nacht bei Raumtemperatur stehengelassen. Anschließend wurden 150 µl 5 M Natriumchlorid hinzugefügt, um die Lösung auf eine Gesamtsalzkonzentration von ca. 0.5 M zu bringen. Die Reaktionsmischung wurde auf eine Kationenaustauschsäule (Pharmacia Mono S HR 5/5) geladen und bei konstantem Gehalt an 25 mM HEPES (pH 7.3) mit einem Salzgradienten von 0.6 M bis 3 M Natriumchlorid fraktioniert. Ca. 15 % gp120 eluierten im Durchfluß; die Hauptmenge an Konjugaten eluierte zwischen 1.2 M und 1.7 M Salzkonzentration und wurde in zwei Fraktionen gepoolt. Diese ergaben nach zweifacher Dialyse gegen 25 mM HEPES (pH 7.3) in der Reihenfolge ihrer Elution eine Fraktion A mit einem Gehalt an 0.7 mg (5.8 nmol) gp120, modifiziert mit 11 nmol Polylysin, und eine Fraktion B mit einem Gehalt an 0.8 mg (6.7 nmol) gp120, modifiziert mit 20 nmol Polylysin. Die Gesamtausbeute dieser Konjugate, bezogen auf gp120 betrug ca. 42 %, bezogen auf Polylysin ca. 65 %.

### Beispiel 5

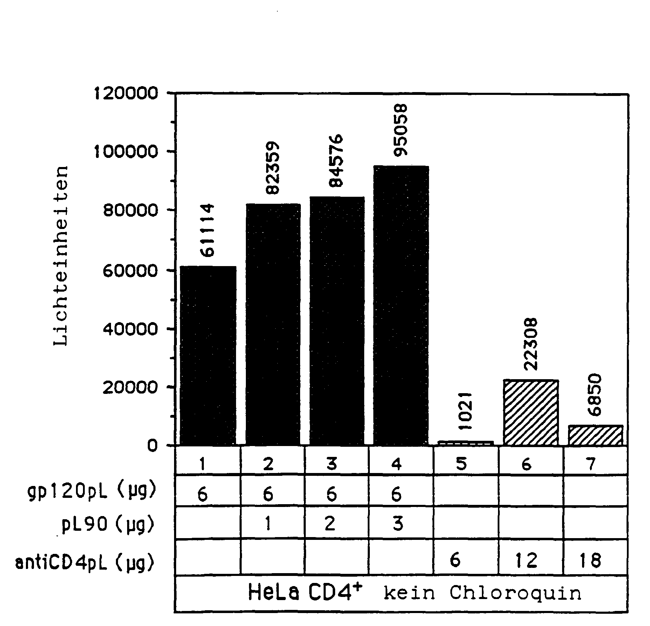

CD4+ HeLa-Zellen (Maddon et al., 1986) in DME-Medium plus 10 % FCS wurden zu 5x10⁵ Zellen pro T25-Flasche ausgesät und anschließend 5 h lang bei 37°C gezüchtet. Die Zellen wurden mit den im Beispiel 4 hergestellten gp120-Polylysin-Konjugaten, komplexiert mit 6 µg pRSVL-DNA, transfiziert; einige der Komplexe enthielten nicht-kovalent gebundenes Polylysin90. Nach 4stündigem Kontakt in Gegenwart von Serum wurde Serum enthaltendes Medium zugesetzt und die Zellen nach 20 h geerntet. Aus den Zellextrakten wurden Aliquots, standardisiert auf gleichen Proteingehalt, auf Luciferase-Aktivität untersucht. Das Ergebnis dieser Versuche ist in Fig. 5 dargestellt; zum Vergleich wurde eine Transfektion mit antiCD4-Polylysin-Konjugaten, hergestellt mittels Kopplung über Disulfidgruppen, durchgeführt. Die Luciferase-Aktivität bezieht sich auf 5x10⁵ Zellen.

### Literatur:

Baltimore D., 1988, Nature 335, 395
Butcher M. et al., 1990, J. Biol. Chem. 265, 5862-5868
Ciliberto G. et al., 1985, Cell 41, 531-540
Da Wet J. et al., 1987, Mol. Cell. Biol. 7, 725-737
Dorland L. et al., 1977, FEBS Lett. 77, 15-20
Fennie Ch. und Lasky L.A., 1989, J. Virology 63, 639-646
Green M. et al., 1989, Cell 58, 215-223
Herskowitz I., 1987, Nature 329, 219
Huebers H.A.und Finch C.A., 1987, Physiol. Reviews 67, 520-282
Jung et al., 1981, Biochem. Res. Commun. 101, 599
Kishimoto T. und Tavassoli M., 1986, Anal. Biochem. 153, 324-329
Kurachi K. et al., 1982, Proc. Natl. Acad. Sci. USA 79, 6461-6464
Lapidot M. und Loyter A., 1990, Exp. Cell Res. 189, 241-246
Lasky L.A. et al., 1986, Science 233, 209-212
Lasky L.A. et al., 1987, Cell 50, 975-985
Lisanti M.P. et al., 1990, Proc. Natl. Acad. Sci. USA 87, 7419-7423
Luthman H. und Magnusson G., 1983, Nucl. Acids Res. 11, 1295-1308
MacGillivray R.T.A. et al., 1983, J. Biol. Chem. 258, 3543
Maddon P.J. et al., 1986, Cell 47, 333-348
Malim M. et al, 1989, Cell 58, 205-214
Maniatis T., 1982, Molecular Cloning, Cold Spring Harbor
Morell A.G. et al., 1966, J. Biol. Chem. 241, 3745-3749
Nygren A. et al., 1988, Proc. Natl. Acad. Sci. USA 85, 6543-6546
O'Shannessy D.J. und Hoffman W.L., 1987, Biotechnol. Appl. Biochem. 9, 488-496
Ponder J.P. et al., 1991, Proc. Natl. Acad. Sci. USA 88, 1217-1221
Riordan J.R. et al., 1989, Science 245, 1066-1073
Trono D. et al., 1989, Cell 59, 113-120
Uchida Y. et al., 1977, J. Biochem. 82, 1425-1433
Valerio D. et al., 1984, Gene 31, 147-153
Van Lenten L. und Ashwell G., 1971, J. Biol. Chem. 246, 1889-1894
Wagner E., et al., 1991, Proc. Natl. Acad. Sci. USA 88, 4255-4259
Wood W.I. et al., 1984, Nature 312, 330-337
Whitt M.A. et al., 1990, J. Virology 64, 4907-4913
Zon G., 1988, Pharmaceut. Research 5, No.9, 539-549
Antibodies, A Laboratory Manual, Cold Spring Harbor Laboratory, 1988, herausgegeben von E. Harlow und D. Lane, S. 539
Lehrbuch der pharmazeutischen Technologie, 1984, Verlag Chemie, 5. Aufl., R. Voigt und M. Bornschein
Pharmazeutische Technologie, 1978, Georg Thieme Verlag Stuttgart, herausgegeben von H. Sucker et al.

## Patentansprüche

1. Konjugate mit der Fähigkeit, mit Nukleinsäuren oder Nukleinsäureanalogen in höhere eukaryotische Zellen aufnehmbare Komplexe zu bilden, bestehend aus einem Glykoprotein und einer Nukleinsäure-bindenden Substanz, dadurch gekennzeichnet, daß das Glykoprotein und die Nukleinsäure-bindende Substanz über eine oder mehrere Kohlenhydratketten des Glykoproteins miteinander verbunden sind.

2. Konjugate nach Anspruch 1, dadurch gekennzeichnet, daß der Glykoproteinanteil von einem Glykoprotein abgeleitet ist, das in seiner nativen Form in einer oder mehreren Kohlenhydratketten endständige Sialinsäure(n) aufweist.

3. Konjugate nach Anspruch 2, dadurch gekennzeichnet, daß das Glykoprotein Transferrin ist.

4. Konjugate nach Anspruch 2, dadurch gekennzeichnet, daß das Glykoprotein gp120 ist.

5. Konjugate nach Anspruch 1 oder 2, dadurch gekennzeichnet, daß das Glykoprotein ein, vorzugsweise monoklonaler, Antikörper gegen ein Zelloberflächenprotein der Zielzellen ist.

6. Konjugate nach einem der Ansprüche 1 - 5, dadurch gekennzeichnet, daß die Nukleinsäure-bindende Substanz in ihrer nativen Form eine oder mehrere Gruppierungen aufweist, die zur Reaktion mit der Aldehydfunktion der oxidierten Kohlenhydratgruppe(n) des Glykoproteins befähigt ist.

7. Konjugate nach Anspruch 6, dadurch gekennzeichnet, daß die Nukleinsäure-bindende Substanz eine oder mehrere primäre und/oder sekundäre Aminogruppen aufweist.

8. Konjugate nach Anspruch 7, dadurch gekennzeichnet, daß die Nukleinsäure-bindende Substanz ein polykationisches Polypeptid ist.

9. Konjugate nach Anspruch 8, dadurch gekennzeichnet, daß die Nukleinsäure-bindende Substanz Polylysin ist.

10. Konjugate nach Anspruch 8, dadurch gekennzeichnet, daß die Nukleinsäure-bindende Substanz ein natürliches DNA-bindendes Protein, ausgewählt aus der Gruppe Histone oder Protamine bzw. Analoge oder Fragmente davon, ist.

11. Konjugate nach einem der Ansprüche 8 - 10, dadurch gekennzeichnet, daß die Summe der positiven Ladungen des Polykations etwa 20 - 500 beträgt.

12. Verfahren zur Herstellung von Konjugaten nach einem der Ansprüche 1 - 11, dadurch gekennzeichnet, daß man ein Glykoprotein unter schonenden Bedingungen zu einer im Kohlenhydratanteil eine oder mehrere Aldehydgruppen enthaltenden Form oxidiert und das oxidierte Glykoprotein mit einer Nukleinsäure-bindenden Substanz koppelt, die eine oder mehrere Gruppierungen mit der Fähigkeit enthält, mit der Aldehydfunktion des oxidierten Glykoproteins zu reagieren.

13. Verfahren nach Anspruch 12, dadurch gekennzeichnet, daß man ein Glykoprotein einsetzt, das eine oder mehrere endständige Sialinsäuren enthält.

14. Verfahren nach Anspruch 13, dadurch gekennzeichnet, daß man Transferrin einsetzt.

15. Verfahren nach Anspruch 13, dadurch gekennzeichnet, daß man gp120 einsetzt.

16. Verfahren nach einem der Ansprüche 12 - 15, dadurch gekennzeichnet, daß man das Glykoprotein mit Perjodat oxidiert.

17. Verfahren nach einem der Ansprüche 12 - 16, dadurch gekennzeichnet, daß man als Nukleinsäurebindende Substanz eine Verbindung einsetzt, die eine oder mehrere primäre und/oder sekundäre Aminogruppen aufweist.

18. Verfahren nach Anspruch 17, dadurch gekennzeichnet, daß man ein homologes polykationisches Polypeptid einsetzt.

19. Verfahren nach Anspruch 18, dadurch gekennzeichnet, daß man Polylysin einsetzt.

20. Verfahren nach einem der Ansprüche 17 - 19, dadurch gekennzeichnet, daß man die Kopplung unter reduzierenden Bedingungen in Gegenwart einer Substanz durchführt, die unter für das Glykoprotein schonenden Bedingungen Schiff'sche Basen selektiv reduziert.

21. Verfahren nach Anspruch 20, dadurch gekennzeichnet, daß man die Kopplung in Gegenwart von Natriumcyanoborhydrid vornimmt.

22. Komplexe, die in menschliche oder tierische Zellen aufgenommen werden, enthaltend ein Konjugat aus einem Glykoprotein und einer Nukleinsäure-bindenden Substanz sowie Nukleinsäure oder ein Nukleinsäureanaloges, dadurch gekennzeichnet, daß das Glykoprotein und die Nukleinsäure-bindende Substanz über eine oder mehrere Kohlenhydratketten des Glykoproteins miteinander verbunden sind.

23. Komplexe nach Anspruch 22, dadurch gekennzeichnet, daß sie als Konjugatanteil eines der in den Ansprüchen 1 - 11 definierten Konjugate enthalten.

24. Komplexe nach Anspruch 22 oder 23, dadurch gekennzeichnet, daß sie zusätzlich ein nichtkovalent gebundenes Polykation, das gegebenenfalls identisch ist mit dem Polykation des Konjugats, derart enthalten, daß die durch das Konjugat erzielte Internalisierung und/oder Expression der Nukleinsäure gesteigert wird.

25. Komplexe nach einem der Ansprüche 22 - 24, dadurch gekennzeichnet, daß sie eine inhibierende Nukleinsäure in Form eines Antisense-Oligonukleotids oder Ribozyms bzw. des jeweils dafür codierenden Gens, gegebenenfalls zusammen mit einem Carrier-Gen, enthalten.

26. Komplexe nach Anspruch 25, dadurch gekennzeichnet, daß sie eine virusinhibierende Nukleinsäure enthalten.

27. Komplexe nach Anspruch 25, dadurch gekennzeichnet, daß sie eine Onkogen-inhibierende Nukleinsäure enthalten.

28. Komplexe nach einem der Ansprüche 22 - 24, dadurch gekennzeichnet, daß sie eine therapeutisch oder gentherapeutisch wirksame Nukleinsäure in Form eines Gens oder eines Genabschnitts enthalten.

29. Verfahren zum Einführen von Nukleinsäuren oder Nukleinsäureanalogen in höhere eukaryotische Zellen in vitro oder ex vivo, wobei man aus einem in einen der Ansprüche 1 - 11 definierten Glykoprotein-Konjugat und Nukleinsäure(n), gegebenenfalls in Gegenwart von nicht-kovalent gebundenem Polykation, einen der in den Ansprüchen 22 - 28 definierten, vorzugsweise unter physiologischen Bedingungen löslichen, Komplexe bildet und Zellen, die ein Zelloberflächenprotein exprimieren, an das das Glykoprotein bindet, mit diesem Komplex in Berührung bringt.

30. Verfahren nach Anspruch 29, dadurch gekennzeichnet, daß man aus einem Transferrin-Konjugat und Nukleinsäure einen Komplex bildet und für die Transfektion der Zellen Bedingungen schafft, unter denen die Transferrin-Rezeptorzahl erhöht wird.

31. Verfahren nach Anspruch 30, dadurch gekennzeichnet, daß man die Transfektion in Gegenwart von Desferrioxamin durchführt.

32. Verfahren nach Anspruch 29, dadurch gekennzeichnet, daß man die Zellen mit dem Komplex unter Bedingungen in Berührung bringt, unter denen der Abbau von Nukleinsäure in der Zelle inhibiert wird.

33. Verfahren nach Anspruch 32, dadurch gekennzeichnet, daß man die Zellen mit Chloroquin behandelt.

34. Pharmazeutische Zubereitung, enthaltend als wirksame Komponente eine oder mehrere therapeutisch oder gentherapeutisch wirksame Nukleinsäuren in Form eines der in den Ansprüchen 22 - 28 definierten Komplexe.

## Claims

1. Conjugates capable of forming, with nucleic acids or nucleic acid analogues, complexes which can be absorbed into higher eukaryotic cells, consisting of a glycoprotein and a nucleic acid-binding substance, characterised in that the glycoprotein and the nucleic acid-binding substance are linked together via one or more carbohydrate chains of the glycoprotein.

2. Conjugates according to claim 1, characterised in that the glycoprotein component is derived from a glycoprotein which in its native form has sialic acid or acids in the terminal position in one or more carbohydrate chains.

3. Conjugates according to claim 2, characterised in that the glycoprotein is transferrin.

4. Conjugates according to claim 2, characterised in that the glycoprotein is gp120.

5. Conjugates according to claim 1 or 2, characterised in that the glycoprotein is a preferably monoclonal antibody against a cell surface protein of the target cells.

6. Conjugates according to one of claims 1 to 5, characterised in that the nucleic acid-binding substance in its native form has one or more groupings which are capable of reacting with the aldehyde function of the oxidised carbohydrate group or groups of the glycoprotein.

7. Conjugates according to claim 6, characterised in that the nucleic acid-binding substance has one or more primary and/or secondary amino groups.

8. Conjugates according to claim 7, characterised in that the nucleic acid-binding substance is a polycationic polypeptide.

9. Conjugates according to claim 8, characterised in that the nucleic acid-binding substance is polylysine.

10. Conjugates according to claim 8, characterised in that the nucleic acid-binding substance is a natural DNA-binding protein selected from the group comprising histones or protamines or analogues or fragments thereof.

11. Conjugates according to one of claims 8 to 10, characterised in that the sum of the positive charges of the polycation is about 20 to 500.

12. Process for preparing conjugates according to one of claims 1 to 11, characterised in that a glycoprotein is oxidised, under mild conditions, into a form which contains one or more aldehyde groups in the carbohydrate portion and the oxidised glycoprotein is coupled with a nucleic acid-binding substance which contains one or more groupings capable of reacting with the aldehyde function of the oxidised glycoprotein.

13. Process according to claim 12, characterised in that a glycoprotein is used which contains one or more terminal sialic acids.

14. Process according to claim 13, characterised in that transferrin is used.

15. Process according to claim 13, characterised in that gp120 is used.

16. Process according to one of claims 12 to 15, characterised in that the glycoprotein is oxidised with periodate.

17. Process according to one of claims 12 to 16, characterised in that as nucleic acid-binding substance is used a compound which has one or more primary and/or secondary amino groups.

18. Process according to claim 17, characterised in that a homologous polycationic polypeptide is used.

19. Process according to claim 18, characterised in that polylysine is used.

20. Process according to one of claims 17 to 19, characterised in that the coupling is carried out under reducing conditions in the presence of a substance which selectively reduces Schiff's bases under conditions which are mild for the glycoprotein.

21. Process according to claim 20, characterised in that the coupling is carried out in the presence of sodium cyanoborohydride.

22. Complexes which are absorbed into human or animal cells, containing a conjugate of a glycoprotein and a nucleic acid-binding substance as well as nucleic acid or a nucleic acid analogue, characterised in that the glycoprotein and the nucleic acid-binding substance are linked together via one or more carbohydrate chains of the glycoprotein.

23. Complexes according to claim 22, characterised in that they contain as conjugate component one of the conjugates as defined in claims 1 to 11.

24. Complexes according to claim 22 or 23, characterised in that they additionally contain a non-covalently bound polycation which may optionally be identical to the polycation of the conjugate, so that the internalisation and/or expression of the nucleic acid achieved by the conjugate is increased.

25. Complexes according to one of claims 22 to 24, characterised in that they contain an inhibiting nucleic acid in the form of an antisense oligonucleotide or ribozyme or the gene coding therefor, optionally together with a carrier gene.

26. Complexes according to claim 25, characterised in that they contain a virus inhibiting nucleic acid.

27. Complexes according to claim 25, characterised in that they contain an oncogene-inhibiting nucleic acid.

28. Complexes according to one of claims 22 to 24, characterised in that they contain a therapeutically or gene therapeutically active nucleic acid in the form of a gene or gene section.

29. Process for introducing nucleic acids or nucleic acid analogues into higher eukaryotic cells *in vitro* or *ex vivo*, in which one of the complexes defined in claims 22 to 28, which is preferably soluble under physiological conditions, is formed from a glycoprotein conjugate as defined in one of claims 1 to 11 and nucleic acid or acids, optionally in the presence of non-covalently bound polycation, and cells which express a cell surface protein to which the glycoprotein binds are brought into contact with this complex.

30. Process according to claim 29, characterised in that a complex is formed from a transferrin conjugate and nucleic acid and conditions are created for the transfection of the cells under which the number of transferrin receptors is increased.

31. Process according to claim 30, characterised in that the transfection is carried out in the presence of desferrioxamine.

32. Process according to claim 29, characterised in that the cells are brought into contact with the complex under conditions in which the breakdown of nucleic acid in the cell is inhibited.

33. Process according to claim 32, characterised in that the cells are treated with chloroquine.

34. Pharmaceutical preparation containing as active component one or more therapeutically or gene therapeutically active nucleic acids in the form of one of the complexes defined in claims 22 to 28.

## Revendications

1. Conjugués ayant la capacité de former avec des acides nucléiques ou des analogues d'acides nucléiques des complexes pouvant être absorbés dans des cellules eucaryotes supérieures, consistant en une glycoprotéine et une substance liant les acides nucléiques, caractérisés en ce que la glycoprotéine et la substance liant les acides nucléiques sont liées l'une à l'autre par une ou plusieurs chaînes glucidiques de la glycoprotéine.

2. Conjugués selon la revendication 1 caractérisés en ce que la partie glycoprotéine dérive d'une glycoprotéine qui, dans sa forme native, présente un ou des acides sialiques terminaux dans une ou plusieurs chaînes glucidiques.

3. Conjugués selon la revendication 2 caractérisés en ce que la glycoprotéine est la transferrine.

4. Conjugués selon la revendication 2 caractérisés en ce que la glycoprotéine est gp120.

5. Conjugués selon la revendication 1 ou 2 caractérisés en ce que la glycoprotéine est un anticorps, de préférence monoclonal, dirigé contre une protéine de la surface cellulaire des cellules cibles.

6. Conjugués selon l'une des revendications 1 - 5 caractérisés en ce que la substance liant les acides nucléiques présente dans sa forme native un ou plusieurs groupements qui sont capables de réagir avec la fonction aldéhyde du ou des groupes glucidiques oxydés de la glycoprotéine.

7. Conjugués selon la revendication 6 caractérisés en ce que la substance liant les acides nucléiques présente un ou plusieurs groupes amino primaires et/ou secondaires.

8. Conjugués selon la revendication 7 caractérisés en ce que la substance liant les acides nucléiques est un polypeptide polycationique.

9. Conjugués selon la revendication 8 caractérisés en ce que la substance liant les acides nucléiques est la polylysine.

10. Conjugués selon la revendication 8 caractérisés en ce que la substance liant les acides nucléiques est une protéine liant les ADN naturelle choisie dans le groupe des histones ou des protamines ou de leurs analogues ou fragments.

11. Conjugués selon l'une des revendications 8 - 10 caractérisés en ce que la somme des charges positives du polycation est d'environ 20 - 500.

12. Procédé de préparation de conjugués selon l'une des revendications 1 - 11 caractérisé en ce que l'on oxyde une glycoprotéine dans des conditions douces en une forme contenant un ou plusieurs groupes aldéhyde dans la partie glucidique et on couple la glycoprotéine oxydée avec une substance liant les acides nucléiques qui contient un ou plusieurs groupements ayant la capacité de réagir avec la fonction aldéhyde de la glycoprotéine oxydée.

13. Procédé selon la revendication 12 caractérisé en ce que l'on utilise une glycoprotéine qui contient un ou plusieurs acides sialiques terminaux.

14. Procédé selon la revendication 13 caractérisé en ce que l'on utilise la transferrine.

15. Procédé selon la revendication 13 caractérisé en ce que l'on utilise gp120.

16. Procédé selon l'une des revendications 12 - 15 caractérisé en ce que l'on oxyde la glycoprotéine avec un periodate.

17. Procédé selon l'une des revendications 12 - 16 caractérisé en ce que l'on utilise comme substance liant les acides nucléiques un composé qui présente un ou plusieurs groupes amino primaires et/ou secondaires.

18. Procédé selon la revendication 17 caractérisé en ce que l'on utilise un polypeptide polycationique homologue.

19. Procédé selon la revendication 18 caractérisé en ce que l'on utilise la polylysine.

20. Procédé selon l'une des revendications 17 - 19 caractérisé en ce que l'on réalise le couplage dans des conditions réductrices en présence d'une substance qui réduit sélectivement les bases de Schiff dans des conditions douces pour la glycoprotéine.

21. Procédé selon la revendication 20 caractérisé en ce que l'on réalise le couplage en présence de cyanoborohydrure de sodium.

22. Complexes qui sont absorbés dans les cellules humaines ou animales, contenant un conjugué d'une glycoprotéine et d'une substance liant les acides nucléiques ainsi qu'un acide nucléique ou un analogue d'acide nucléique, caractérisés en ce que la glycoprotéine et la substance liant les acides nucléiques sont liées l'une à l'autre par une ou plusieurs chaînes glucidiques de la glycoprotéine.

23. Complexes selon la revendication 22 caractérisés en ce qu'ils contiennent comme partie conjugué l'un des conjugués définis dans les revendications 1 - 11.

24. Complexes selon la revendication 22 ou 23 caractérisés en ce qu'ils contiennent en outre un polycation lié de manière non covalente, qui est éventuellement identique au polycation du conjugué, de telle manière que l'internalisation et/ou l'expression de l'acide nucléique réalisées par le conjugué sont augmentées.

25. Complexes selon l'une des revendications 22 - 24 caractérisés en ce qu'ils contiennent un acide nucléique inhibiteur sous forme d'un oligonucléotide antisens ou d'un ribozyme ou du gène codant chacun d'eux, éventuellement en même temps qu'un gène porteur.

26. Complexes selon la revendication 25 caractérisés en ce qu'ils contiennent un acide nucléique inhibant des virus.

27. Complexes selon la revendication 25 caractérisés en ce qu'ils contiennent un acide nucléique inhibant des oncogènes.

28. Complexes selon l'une des revendications 22 - 24 caractérisés en ce qu'ils contiennent un acide nucléique efficace du point de vue thérapeutique ou de la thérapie génique sous forme d'un gène ou d'un segment de gène.

29. Procédé pour introduire des acides nucléiques ou des analogues d'acides nucléiques dans des cellules eucaryotes supérieures in vitro ou ex vivo où l'on forme à partir d'un conjugué de glycoprotéine défini dans l'une des revendications 1 - 11 et d'acide(s) nucléique(s), éventuellement en présence d'un polycation lié de manière non covalente, l'un des complexes définis dans les revendications 22 - 28, de préférence solubles dans les conditions physiologiques, qui expriment une protéine de la surface cellulaire, et on met en contact avec ce complexe des cellules qui expriment une protéine de la surface cellulaire à laquelle se lie la glycoprotéine.

30. Procédé selon la revendication 29 caractérisé en ce que l'on forme un complexe à partir d'un conjugué de transferrine et d'acide nucléique et on crée pour la transfection des cellules des conditions dans lesquelles le nombre de récepteurs de la transferrine est augmenté.

31. Procédé selon la revendication 30 caractérisé en ce que l'on réalise la transfection en présence de desferrioxamine.

32. Procédé selon la revendication 29 caractérisé en ce que l'on met en contact les cellules avec le complexe dans des conditions dans lesquelles la dégradation des acides nucléiques dans la cellule est inhibée.

33. Procédé selon la revendication 32 caractérisé en ce que l'on traite les cellules avec la chloroquine.

34. Préparation pharmaceutique contenant comme composant efficace un ou plusieurs acides nucléiques efficaces du point de vue thérapeutique ou de la thérapie génique sous forme de l'un des complexes définis dans les revendications 22 - 28.
